Europäisches Patentamt

European Patent Office

Office européen des brevets

(11) Publication number: **0 355 013**
**A2**

# EUROPEAN PATENT APPLICATION

(21) Application number: 89114783.7

(22) Date of filing: 10.08.89

(51) Int. Cl.⁴: **G01N 33/543 , G01N 33/68 , G01N 33/577**

(30) Priority: 18.08.88 US 236109

(43) Date of publication of application:
21.02.90 Bulletin 90/08

(84) Designated Contracting States:
AT CH DE ES FR GB IT LI

(71) Applicant: F. HOFFMANN-LA ROCHE AG
Postfach 3255
CH-4002 Basel(CH)

(72) Inventor: Familleti, Philip C.
24 Clover Hill Road
Millington, N.J. 07946(US)
Inventor: Wardell-Swanson, Judith A.
14 Lake Shore Drive
Lake Hiawatha, N.J. 07034(US)

(74) Representative: Kloter, Rudolf et al
Postfach 3255 Grenzacherstrasse 124
CH-4002 Basel(CH)

(54) Immunobioassay.

(57) This invention provides a novel immunobioassay, through the use of which biologically active proteins and polypeptides can be quantified in biological fluids and laboratory samples without interference from substances in the samples which can adversely effect standard bioassays.

EP 0 355 013 A2

## Immunoassay

This invention relates to a novel immunobioassay for the quantitation of the bioactivity of biologically active proteins and polypeptides in biological fluids or laboratory samples.

The availability of a growing number of biologically active proteins and polypeptides from natural sources or from the application of recombinant DNA technology has led to new therapeutic approaches in human and veterinary medicine. Proper clinical evaluation of such proteins and polypeptides requires accurate quantitation of endogeneous levels and of levels attained following their administration. During the isolation of such proteins and polypeptides there is also a need for reliable ways to quantify bioactivity at the various stages of purification and in the final preparations.

Frequently, the levels of biologically active proteins and polypeptides, in biological fluid samples taken from human or animal subjects are measured by bioassay. For example Gillis et al. [J. Immunol. 120:2027 (1978)] have developed a bioassay for T cell growth factor (IL-2) in which the incorporation of $^3$H-thymidine into DNA in murine cytotoxic lymphoid lines (CTLL) in response to growth stimulation by IL-2 is quantified. More recently, Familletti et al. (U.S. Patent application serial No. 06/676,453) have disclosed a colorimetric variation of the Gillis et al. assay in which the production of lactic acid by CTLL cells is the measured as indicator of growth stimulation.

Unfortunately, serum and other biological fluids frequently contain materials that can adversely affect bioassays, causing measurements to be either too low or too high. There may, for example, be inhibitory substances that depress the true values. Conversely, there may be other factors having similar biological activities which produce a greater response in the assay cells than can properly be attributed to the protein or polypeptide of interest.

Even laboratory samples can contain substances that can prevent accurate bioassay measurements. For example, Orosz et al. [J. Immunol. 130:1764 (1983)] have reported that phorbol myristate acetate (PMA) and other agents used to induce lymphokine production in cultured cells can interfere with various bioassays used to quantify lymphokines such as IL-1, IL-2 and IL-3.

In an effort to avoid the effects of interfering sample materials, some investigators have turned from bioassays to immunoassays. For instance, Gehman et al. [J. Immunol. Meth. 74:39 (1984)] have developed an enzyme-linked immunosorbant assay (ELISA) for quantifying human IL-2. Substances in human serum and mitogens such as PMA and phytohemagglutinin are said to not interfere with measurements made with this assay. Similarly, Budd et al. [Bio/Technology 4:983 (1986)] have developed monoclonal antibody-based ELISA and radioimmunoassays for IL-2.

But the problem with immunoassays is that they do not always reflect true bioactivity. Antibodies will simply bind to the epitopes against which they are directed, even if the epitopes are contained in a wholly denatured, inactive protein, or polypeptide, or in a small degradation fragment of the active molecule.

This invention provides a method for the assay of biologically active polypeptides and proteins (hereinafter collectively referred to as "proteins") which is highly specific and sensitive and which avoids the problems caused by interfering substances in biological fluids and laboratory samples.

More particularly, this invention provides a method for quantifying the amount of a biologically active protein in a sample, comprising:

(a) contacting a biological fluid or laboratory sample suspected to contain a biologically active protein with an immobilized antibody specific for the protein under conditions in which the antibody binds specifically to the protein at a site that will not impair activity, to produce an antibody-protein complex;

(b) washing the antibody-protein complex with a solution to remove interfering substances present in the sample;

(c) contacting the washed antibody-protein complex with a cell capable of responding to the biologically active protein to produce a measurable response; and

(d) measuring the response produced, thereby quantifying the amount of the biologically active protein.

The method of this invention can be used to detect and quantify biologically active proteins in a wide variety of biological fluids and laboratory samples. Biological fluids that can be assayed include, e.g., blood, serum, lymph, seminal fluid, milk, saliva and other secretory fluids. Assayable laboratory samples include, e.g., conditioned media from the culture of various primary explants and secondary cultures or established cell lines of avian, mouse, human or other animal origins and purification step fractions from such media or from one of the biological fluids mentioned above.

The method of the invention involves a basic two-step process. First, the biologically active protein in an aliquot from a biological fluid or laboratory sample is "captured" by an antibody, and extraneous materials that could either enhance or inhibit the activity of the protein are removed by washing. As used herein, the word "aliquot" is taken to mean a small representative portion of the sample, typically from about 25 to about 100 μl in volume. Then the protein still bound to the antibody is contacted with a cell capable of responding to the protein.

Suitable antibodies include polyclonal and monoclonal antibodies which are capable of binding to the protein of interest. To produce antibodies against a biologically active protein, the protein is first isolated using standard methods from a convenient source such as from a biological fluid, tissue, cell culture conditioned medium or recombinant expression system and then preferably purified using conventional protein purification techniques including but not limited to gel filtration, ion-exchange chromatography, preparative disc-gel or curtain electrophoresis, isoelectric focusing, low temperature organic solvent fractionation, salt precipitation (using e.g., sodium or ammonium sulfate) or countercurrent distribution.

After purification, the protein can used directly as a vaccine to elicit the production of antibodies in a mammalian or avian animal by injecting an effective amount of the protein. The appropriate amount of the protein which would be required would be known to those skilled in the art or could readily be determined by routine experimentation.

Alternatively, and preferably, the protein is first modified to enhance immunogenicity.

Enhanced immunity can be produced in one of two ways. First, an adjuvant or immunopotentiator can be added to the vaccine. Secondly, the proteins can be presented to an animal that is to be immunized in a larger form, either as a cross-linked complex or conjugated to a carrier molecule.

Suitable adjuvants for the vaccination of animals include but are not limited to Freund's complete or incomplete adjuvant (not suitable for human or livestock use), Adjuvant 65 (containing peanut oil, mannide monooleate and aluminum monostearate); mineral gels such as aluminum hydroxide, aluminum phosphate and alum; surfactants such as hexadecylamine, octadecylamine, lysolecithin, dimethyldioctadecylammonium bromide, N,N-dioctadecyl-N′,N′-bis (2-hydroxymethyl) propanediamine, methoxyhexadecylglycerol and pluronic polyols; polyanions such as pyran, dextran sulfate, poly IC, polyacrylic acid and carbopol; peptides such as muramyl dipeptide, dimethylglycine and tuftsin; and oil emulsions. The proteins could also be administered following incorporation into liposomes or other microcarriers. Incorporation into liposomes or other microcarriers provides a means by which the release of the vaccines can be sustained over a prolonged period of time.

The immunogenicity of the proteins, especially the smaller ones, can be enhanced by cross-linking or by coupling to an immunogenic carrier molecule (i.e., a macromolecule having the property of independently eliciting an immunological response in a host animal, to which the proteins can be covalently linked). Cross-linking or conjugation to a carrier molecule may be required because small proteins sometimes act as haptens (molecules which are capable of specifically binding to an antibody but incapable of eliciting antibody production, i.e., they are not immunogenic). Conjugation of such small proteins to an immunogenic carrier molecule renders them immunogenic through what is commonly known as the "carrier effect".

Suitable carrier molecules include, e.g., proteins and natural or synthetic polymeric compounds such as polypeptides, polysaccharides, lipopolysaccharides etc. A useful carrier is a glycoside called Quil A, which has been described by Morein et al., Nature 308:457 (1984). Protein carrier molecules are especially preferred, including but not limited to mammalian serum proteins such as keyhole limpet hemocyanin, human or bovine gammaglobulin, human, bovine or rabbit serum albumin, or methylated or other derivatives of such proteins. Other protein carriers will be apparent to those skilled in the art. Preferably, but not necessarily, the protein carrier will be foreign to the host animal in which antibodies against the proteins are to be elicited.

Covalent coupling to the carrier molecule can be carried out using methods well known in the art, the exact choice of which will be dictated by the nature of the carrier molecule used. When the immunogenic carrier molecule is a protein, the biologically active proteins of the invention can be coupled, e.g., using water soluble carbodiimides such as dicyclohexylcarbodiimide, or glutaraldehyde.

Coupling agents such as these can also be used to cross-link the proteins to themselves without the use of a separate carrier molecule. Such cross-linking into protein aggregates can also increase immunogenicity.

Initial vaccinations are preferably followed by booster vaccinations administered from one to several weeks later. Multiple boosters may be administered. Standard routes of administration can be used such as subcutaneous, intradermal, intraperitoneal or intramuscular administration can be used for all injections.

Suitable animals for vaccination include, e.g., large mammalian species such as goats, cows, cattle, sheep or horses; smaller mammalian species such as rabbits, guinea pigs or other rodents; or avian species.

After allowing time for an antibody titer for develop, serum is isolated by standard methods and the IgG fraction is preferably isolated by plasmaphoresis or other conventional means.

Preferably, monoclonal antibodies are used in this invention. To produce monoclonal antibodies, a biologically active protein of interest is used to immunize an animal as described above to obtain antibody-producing cells for fusion to myeloma cells.

Somatic cells having the potential for producing antibody, particularly B cells, are suitable for fusion with a myeloma cell line. These somatic cells may be derived from the lymph nodes, spleens and peripheral blood of primed animals. Preferably mouse spleen cells are used, in part because these cells produce a relatively high percentage of stable fusions with mouse myeloma lines. It would be possible, however, to use rat, rabbit, frog or other cells instead.

Specialized myeloma cell lines have been developed from lymphocytic tumors for use in hyridoma-producing fusion procedures [Kohler and Milstein. Eur. J. Immunol. 6:511 (1976); Shulman et al., Nature 276:269 (1978); Volk et al., J. Virol. 42:220 (1982)]. These cell lines have been developed for at least three reasons. The first is to facilitate the selection of fused hybridomas among unfused and similarly indefinitely self-propogating myeloma cells. Usually, this is accomplished by using myelomas with enzyme deficiencies that render them incapable of growing in certain selective media that support the growth of hybridomas. The second reason arises from the inherent ability of lymphocytic tumor cells to produce their own antibodies. The purpose of using monoclonal techniques is to obtain fused hybrid cell lines with unlimited lifespans that produce the desired single antibody under the genetic control of the somatic cell component of the hybridoma. To eliminate the production of tumor cell antibodies by the hybridomas, myeloma cell lines incapable of producing light or heavy immunoglobulin chains or deficient in antibody secretion mechanisms are preferably used. A third reason for selection of these cell lines is for their suitability and efficiency for fusion.

Many myeloma cell lines may be used for the production of fused cell hybrids, including e.g., P3/X63-Ag 8, P3/NSI/1-Ag 4-1. SP2/0-Ag-14 and S194/5.XXO.BU.1. The P3/X63-Ag 8 and P3/NSI/1-Ag 4-1 cell lines have been described by Kohler and Milstein [Eur. J. Immunol. 6:511, (1976)]. Shulman et al. [Nature 276:269 (1978)] developed the Sp2/0-Ag14 myeloma line. The S194/5.XXO.BU.1 line was reported by Trowbridge [J. EXP. Med. 148:313 (1979)].

Methods for generating hybrids of antibody-producing spleen or lymph node cells and myeloma cells usually involve mixing somatic cells with myeloma cells in a 10:1 proportion (although the proportion may vary from about 20:1 to about 1:1), respectively, in the presence of an agent or agents (chemical, viral or electrical) that promote the fusion of cell membranes. It is preferred that the same species of animal serve as the source of the somatic and myeloma cells used in the fusion procedure. Fusion methods have been described by Kohler and Milstein [Nature 256:495 (1975) and Eur. J. Immunol. 6:511 (1976)], by Gefter et al. [Somatic Cell Genet. 3:231 (1977)], and by Volk et al. (J. Virol. 42:220 (1982)]. The fusion-promoting agents used by those investigators were Sendai virus and polyethylene glycol.

Because fusion procedures produce viable hybrids at very low frequency (e.g., when spleens are used as a source of somatic cells, only one hybrid is obtained for roughly every $1 \times 10^5$ spleen cells), it is essential to have a means of selecting the fused cell hybrids from the remaining unfused cells, particularly the unfused myeloma cells. A means of detecting the desired antibody-producing hybridomas among other resulting fused cell hybrids is also necessary.

Generally, the selection of fused cell hybrids is accomplished by culturing the cells in media that support the growth of hybridomas but prevent the growth of the myeloma cells, which normally would go on dividing indefinitely. (The somatic cells used in the fusion do not maintain long-term viability in in vitro culture and hence do not pose a problem). Myeloma cells lacking hypoxanthine phosphoribosyl transferase (HPRT-negative) can conveniently be used. Selection against these cells is made in hypoxanthine/aminopterin/thymidine (HAT) medium, a medium in which the fused cell hybrids survive due to the HPRT-positive genotype of the spleen cells. The use of myeloma cells with different genetic deficiencies (drug sensitivities, etc.) that can be selected against in media supporting the growth of genotypically competent hybrids is also possible.

Several weeks are usually required to selectively culture the fused cell hybrids. Early in this time period, it is necessary to identify those hybrids which produce the desired antibody so that they may subsequently be cloned and propagated. Generally, around 10% of hybrids obtained produce the desired antibody, although a range of from 1 to 30% is not uncommon. The detection of antibody-producing hybrids can be achieved by any one of several standard assay methods, including enzyme-linked immunoassay and radioimmunoassay techniques which have been described in the literature [see, e.g., Kennet et al. (editors), Monoclonal Antibodies and Hybridomas: A New Dimension in Biological Analyses, pp. 376-384, plenum press, New York (1980)].

Once the desired fused cell hybrids have been selected and cloned into individual antibody-producing cell lines, each cell line may be propagated in either of two standard ways. A suspension of the hybridoma cells can be injected into a histocompatible animal. The injected animal will then develop tumors that secrete the specific monoclonal antibody produced by the fused cell hybrid. The body fluids of the animal, such as serum or ascites fluid, can be tapped to provide monoclonal antibodies in high concentration. Alternatively, the individual cell lines may be propagated in vitro in laboratory culture vessels. The culture medium containing high concentrations of a single specific monoclonal antibody can be harvested by decantation, filtration or centrifugation.

The antibodies used in this invention can be immobilized by simply coating them onto a suitable substrate such as the wells of a microtiter or enzyme immunoassay plate. Alternatively, they can be covalently coupled using standard methods to an insoluble support such as a cellulose, polyacrylamide, silica gel, agarose or dextran support. Beaded supports such as the supports made by Pharmacia Fine Chemicals or Biorad Laboratories, many of which are available in already activated forms for the coupling of proteins, are especially convenient.

The binding of the biologically active proteins to the antibodies in the immunobioassay of this invention are carried out under standard conditions in which specific binding occurs. These conditions should preferably be such that the amino acid residue charges and the conformations of the antibody and protein are close to what they would be under physiological conditions. Preferably, the immobilized antibodies are first treated with a solution to block the antibodies and the substrate to which they are attached against nonspecific binding. This prevents undesired binding of stimulatory or inhibitory substances in the assay sample.

In the example provided below to illustrate the invention, a solution containing fetal bovine serum, bovine serum albumin and gelatin was used for this purpose. Other blocking agents that could instead be used include but are not limited to ovalbumin and non-fat dry milk.

Once a protein of interest is bound to a complementary antibody, the antibody-protein complex is washed to remove interfering substances. A wide range of wash solutions can be used, such as distilled water, phosphate buffered saline (PBS) or other buffered solutions.

It is necessary for the method of the present invention that the immobilized antibody binds specifically to the protein at a position that will not substantially impair the ability of the protein to produce a response in an appropriate cell.

According to the instant invention a responsive cell is transferred to the container with the washed antibody-protein complex, thereby permitting all steps at the method to be carried out in the same container.

Numerous biologically active proteins and responsive cells can be used in this invention. For example, the immunobioassay can be carried out on leukocyte interferon by measuring its antiproliferative effects on Daudi cells [Evinger et al., J. Biol. Chem. 256:2113 (1980)]. IL-1 can be quantified by measuring its ability to stimulate the growth of various cultured cell lines [Schmidt et al., J. Immunol. 128:2177 (1982)]. As shown in the Example below, IL-2 can be assayed by measuring growth stimulation in CTLL cells, etc. Proteins from natural sources and proteins produced by recombinant DNA techniques in prokaryotic or eukaryotic expression systems are all amenable to immunobioassay.

Any biological response observed is quantified by comparison to a standard curve, prepared by measuring a series dilution of a known amount of the protein prepared in the same medium (e.g., serum or serum-free culture medium).

The following, nonlimiting example will serve to better illustrate this invention.

## EXAMPLE

Materials:

1. Human Serum HumanAB serum was obtained from GIBCO Laboratories (Grand Island, N.Y.).

2. 5B1 antibody Purified monoclonal antibody to non-glycosylated forms of IL-2 was obtained from Richard Chizzonite at Hoffmann-La Roche Inc. (Nutley, N.J.).

3. rIL-2 purified rIL-2 was obtained from Hoffmann-La Roche Inc. (Nutley, N.J.).

4. HB101 assay media serum free assay media HB101 was obtained from Hana Biologics (Alameda, CA) and prepared as directed.

5. RPMI 1640 cell maintenance media CTLL cells were maintained in RPMI 1640 obtained from GIBCO (Grand Island. N.Y.).

6. CTLL cells CTLL cells used in the assay were obtained from the American Type Culture Collection.

7. Immulon II EIA plates EIA plates used in the assay are available from Dynatech Corp. (Chantilly, Va).

8. ELISA plate reader AnArtek vertical beam spectrophotometer was used to determine Optical Densities.

9. Cetus Propette All plate filling, serial dilutions and plate-to-plate transfers were acomplished with the aid of an automatic pipeting device obtained from the Cetus Corp. (Emeryville, Ca).

Procedure:

1. IL-2 capture method ELISA plates were coated with antibody to IL-2 by first diluting the 5B1 antibody in 50mM Tris, 250mM NaCl, PH 8.0 (filter sterilized), to a final concentration of 20 $\mu$g/ml. 100 $\mu$l of the diluted antibody was added to each well of a 96-well Immulon II EIA plate. The plate was sealed with an adhesive-backed plate sealer and allowed to incubate overnight at room temperature. The following day, the plates were washed 3 times with distilled $dH_2O$. After washing, the plates were blocked by adding 100 $\mu$l of 10% Fetal Bovine serum, 1% BSA, 0.3% gelatin, 25mM NaPO4, filter sterilized and prewarmed to 37°C. The plates were incubated for 30 minutes at 37°C and then washed 3 times with sterile PBS. The plate was filled with 100 $\mu$l/well of Fetal Bovine Serum and was then ready for sample addition. Three samples were prepared to simulate clinical serum samples. Purified recombinant IL-2 was added to three aliquots of normal human serum to a achieve final concentrations of 200 units/ml, 20 units/ml and 2 units/ml, respectively. 100 $\mu$l of a sample was added to the first well of a row on the EIA plate. The final volume in the first well of each row was 200 $\mu$l. After each sample had been added rhe plate was serially diluted across all 12 wells of each row, mixing 4 times between each transfer. Each transfer contained 100 $\mu$l and represented a 1:2 dilution. The IL-2 was allowed to bind to the coated plate for two hours at room temperature on an orbital shaker. Following the binding step, the plates were washed 5 times with sterile PBS.

2. The IL-2 bioassay CTLL cells were prepared for bioassay by centrifuging the cells at 500 x g to remove residual IL-2 and serum containing growth media. The cell pellet was resuspended in HB101 assay media and centrifuged as before. After three washes, the cell pellet was resuspended in HB101 at a concentration of $1 \times 10^5$ cells/ml. 200 $\mu$l of cell suspension was added to the washed EIA plate. The plate was covered with a non-airtight lid and allowed to incubate at 37°C in a 5% $CO_2$ incubator for 48 hrs.

3. Determining IL-2 dependent cellular prolifertion IL-2 dependent proliferation was quantified by the colorimetric analysis of lactic acid production. Lactic acid is a secondary metabolite of glucose consumption and is an acurate indicator of IL-2-dependent cellular proliferation. Plates were prepared for the colorimetric reaction by adding 50 $\mu$l of solution A to each well of a fresh Immulon II plate. 6 ml of solution A was prepared by resuspending 10 mg of NAD (Sigma #260-110) with 2.0 ml of glycine buffer (Sigma #826-3), 0.2 ml 1% gelatin, and 4.0 ml of sterile distilled $dH_2O$. A 15 $\mu$l sample from each well of the bioassay plate was then transferred to the corresponding well on the colorimetric reaction plate (containing solution A). A timed color reaction was initiated with the addition of 50 $\mu$l of solution B to each well of the color reaction plate. Solution B was prepared immediately before addition to by combining 5.0 ml of p-iodonitrotetrazolium violet (INT, Sigma #940-5) and 1.0 ml of phenazine methosulfate (PMS, 0.02 mg/ml in $H_2O$, Sigma #P9625) and 0.1 ml of lactic dehyrogenase (LDH, Sigma #826-6). The color reaction was stopped after a 10 minute incubation at room temperature with the addition of 50 $\mu$l/well of 0.35M HCl. The optical densities were determined with the aid of an ELISA plate reader with a 550nm filter.

Results:

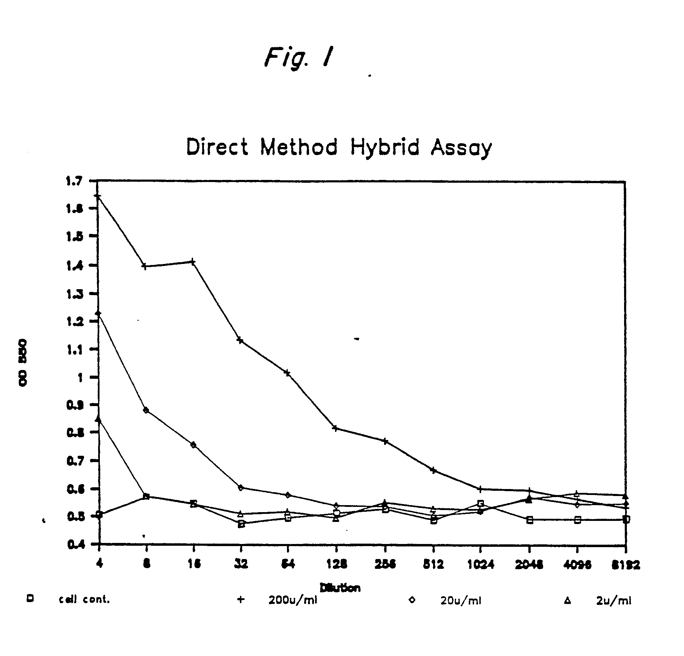

The O.D. 550 obtained from the three simulated clinical samples were plotted and are shown in Fig. 1. Also shown is the plot of the O.D. 550 obtained from a blank sample containing NHS but no IL-2. As indicated by these plots. IL-2 captured by the 5BI antibody can be quantitated. As little as 2 units of IL-2 per ml of Human serum was detected in the assay.

Figur 1 is a graphical representation of an immunobioassay of reombinant human IL-2 showing absorbance at 550 $\mu$m as a function of sample dilution for the indicated samples.

**Claims**

1. A method for quantifying the amount of a biologically active protein in a sample, comprising:
(a) contacting an aliquot of a biological fluid or laboratory sample suspected to contain a biologically active protein with an immobilized antibody specific for the protein under conditions in which the antibody binds specifically to the protein at a site that will not substantially impair the bioactivity of the protein, to produce an antibody-protein complex;
(b) washing the antibody-protein complex with a solution to remove interfering substances present in the sample;
(c) contacting the washed antibody-protein complex with a cell capable of responding to the biologically active protein to produce a measurable response; and
(d) measuring the response produced, thereby quantifying the amount of the biologically active

active protein.

2. A method of claim 1 in which the antibody is from an antiserum.

3. A method of claim 1 in which the antibody is a monoclonal antibody.

4. A method according to claims 1 to 3 in which the biologically active protein is IL-2 and the responsive cell is a CTLL cell.

5. The method of claim 4 in which the response of the CTLL cell is measured by the colorimetric determination of lactic acid production.

6. The method of claim 4 in which the response of the CTLL cell is measured by the determination of the incorporation $^3$H-thymidine into DNA.

7. A method according to claims 1 to 6 in which all steps are carried out in the same container.

Fig. 1

Direct Method Hybrid Assay